# EUROPEAN PATENT APPLICATION

(11) **EP 2 402 027 A1**
(43) Date of publication of application: **04.01.2012**
(21) Application number: 10305714.7
(22) Date of filing: 01.07.2010
(51) Int. Cl.: A61K 39/125, C07K 14/085, A61P 3/10

(54) **Enterovirus vaccines for preventing or treating type 1 diabetes (I)**

(71) Applicant: Sanofi Pasteur, 69007 Lyon Cedex (FR); Vactech OY, 33520 Tampere (FI)
(72) Inventor: Knip, Mickael, 00130, HELSINKI (FI); Hyöty, Heikki, 33230, TAMPERE (FI); Laitinen, Olli, 33270, TAMPERE (FI); Tolonen, Outi, 90120, OULU (FI); Pulkki, Minna, 33560, TAMPERE (FI); Oikarinen, Sami, 33500, TAMPERE (FI); Honkanen, Hanna-Riikka, 33520, TAMPERE (FI); Lecouturier, Valérie, 69380, CHAZAY d'AZERGUES (FR); Almond, Jeffrey, 69570, DARDILLY (FR)
(74) Representative: Boije-Backman, Solveig Magdalena

(57) **Abstract**

Some coxsackie B viruses have been found to have a protective effect on type 1 diabetes, while others increase the risk of the disease. This opens up new therapeutic implications in the form of vaccines. The protective viruses CBV3 and CBV6 may be used in live attenuated vaccines for preventing or treating type 1 diabetes. They may also be used for inducing an immune response against risk viruses like CBV1, and for eliminating the diabetogenic effect of the risk viruses. Type 1 diabetes may be prevented especially by vaccinating with live attenuated protective viruses CBV3 and CBV6, and further with inactivated diabetogenic/risk virus CBV1, components thereof or antibodies thereto.

## Description

### Field of the Invention

The present invention relates to vaccines for prevention and treatment of type 1 diabetes. Especially the invention relates to vaccines comprising live attenuated coxsackie B viruses found to have a protective effect against the disease.

### Background of the Invention

Type 1 diabetes (T1 D) is a severe disease that has become more and more frequent already at a very early age. In type 1 diabetes the beta-cells of the pancreas are destroyed, which leads to insulin deficiency. The destruction of the beta-cells is believed to be caused by an autoimmune response, which in turn is assumed to be induced by a virus infection.

The connection between enteroviruses and type 1 diabetes (T1D) has been documented in a multitude of studies. Enteroviruses have been detected in the pancreas, blood and intestinal mucosa of patients with type 1 diabetes more frequently than in control subjects and prospective studies have supported their role in the initiation of the beta-cell damaging process associated with type 1 diabetes. Enteroviruses infect pancreatic beta-cells in cell culture and cause diabetes in animal models. Moreover, recent studies indicate that the genetic susceptibility to type 1 diabetes is modulated by a polymorphism in the *IFIH1* gene which encodes an innate immune system receptor for enteroviruses. This evidence has generated increasing demands to develop vaccines against diabetogenic enteroviruses. However, it is not known which enterovirus serotypes are involved and their identification would be needed for the development of effective vaccines.

The group of enteroviruses includes more than 100 different serotypes. Enterovirus infections are usually subclinical, but they may also cause various kinds of diseases. For example polioviruses, coxsackie B virus (CBV), coxsackie A virus (CAV), and echovirus (E, or Echo) as well as numbered enteroviruses are enteroviruses known to be involved in the development of a variety of diseases. The spectrum of responsible serotypes varies a lot from disease to disease, and even in one disease like type 1 diabetes the exact serotypes have not been fully and reliably identified.

Indeed, previous studies reporting association between enteroviruses and T1D were not able to discriminate between serotype or were restricted to case reports. Some studies have suggested that CBV serotypes may be important (Yoon et al, 1979 New Eng J of Med, v300, p1173; Hindersson M, et al., 2005, J Clin Virol v.33 p158; Dotta F. et al., 2007, PNAS 104: 5115). However, other enterovirus serotypes have also been sporadically reported (Cabrera-Rode et al., 2003, Diabetologia; Williams et al., 2006, J Clin Micro v.44 p441).

WO01/00236 (Hyöty et al.) suggests the use of oral poliovirus in a vaccine against type 1 diabetes. In addition it suggests another diabetes vaccine comprising one or more inactivated non-polio enteroviruses selected from the group consisting of CBV serotypes 1, 2, 3, 4, 5 and 6, echovirus serotypes 3, 4, 6, 9, 11, 22 and 30, CAV serotypes 9 and 16. However, no data is given as to the role of the individual serotypes listed, except for some animal tests with CBV3. A more recent publication US2010/0047273 (Rappuoli et al.) suggests the use of CBV4, and in particular of a Tuscany strain thereof for preventing or treating type 1 diabetes.

Still there is a great need for developing effective means and methods for preventing and treating type 1 diabetes. The present invention meets this need.

### Summary of the Invention

Previous publications suggest that there is an association between enteroviruses, and especially between coxsackie B viruses (CBV) and type 1 diabetes. It has been assumed that enteroviruses may induce the development of type 1 diabetes, and that some serotypes are more diabetogenic than others. The present invention resides in the surprising finding that not only are particular serotypes more diabetogenic than others, but in addition there are serotypes that have a protective effect on type 1 diabetes.

Induction of heterotypic virus resistance has previously been reported by Landau B J et al., Microbial Pathogenesis 1990:8 289-298, but not in connection with diabetes. Said publication discloses an increased resistance against liver, heart and pancreas damage caused by CBV1 infection in mice vaccinated with CBV3. CBV3 was found to cause subtotal pancreatic acinar cell damage, while CBV1 caused total acinar cell necrosis. When CBV3 vaccinated mice were challenged with CBV1 no augmentation of existing pancreatic acinar cell damage occurred. Noteworthy, however, was that neither CBV3 nor CBV1 was found to affect the insulin producing islet cells i.e. beta-cells of the pancreas.

The present inventors found that children who had been in contact with CBV3 or CBV6 had a reduced risk of contracting type 1 diabetes, while others who had been in contact with e.g. CBV1 or CBV2 had an increased risk. The finding of the division of enteroviruses into protective and risk serotypes opens new therapeutic implications in the prevention and treatment of type 1 diabetes.

It is striking that among all the serotypes studied the ones which showed the most marked risk or protective effect belonged to the CBV group. This is even more striking when the low number of CBV serotypes (six) is taken into account. This finding makes it is possible to make an effective enterovirus vaccine against type 1 diabetes.

The present invention now provides a live attenuated coxsackie B virus selected from the group consisting of CBV3 and CBV6 for use in preventing or treating type 1 diabetes.

The invention further provides a vaccine comprising live attenuated coxsackie B virus selected from the group consisting of CBV3 and CBV6.

Further, the application discloses a method of preventing or treating type 1 diabetes, of inducing an immune response against a diabetogenic enterovirus, or of eliminating the diabetogenic effect of an enterovirus in a subject in need thereof, comprising vaccinating the subject with at least one live attenuated coxsackie B virus selected from the group consisting of CBV3 and CBV6.

Some preferred embodiments are set forth in the dependent claims.

### Brief Description of the Drawings

Figure 1 shows the OR and Cl values, and the prevalence of antibodies for the CBV serotypes.
Figure 2 shows a summary of the tight and loose criteria of CBV1, CVB3 and CBV6 time window analyses.
Figure 3 shows a combined population attributable risk (PAR) for CBV1 and CBV2.
Figure 4 shows protective PAR% for CBV3 and CBV6 using classes positive vs. negative
Figure 5 shows neutralizing antibodies against CBV serotypes found in a child testing positive for CBV3 in a stool sample.

### Detailed Description of the Invention

CBV3 and CBV6 were found to be associated with a reduced risk of type 1 diabetes, whereas especially CBV1 was associated with an increased risk of the disease. Consequently CBV3 and CBV6 may be classified as protective or non-diabetogenic enteroviruses, whereas CBV1 and CBV2 may be classified as risk or diabetogenic viruses. The non-diabetogenic CBV3 and CBV6 are structurally very close to the diabetogenic CBV1. Thus, a possible explanation is that the immune response induced by CBV3 and CBV6 cross-reacts with CBV1 providing cross-protection. In fact, the inventors found that children who have first been infected with CBV3 or CBV6 are protected against the diabetogenic effect of later exposure to CBV1. Because neutralizing antibodies show only low and transient cross-reactivity between CBV1 and CBV3/CBV6 the biological explanation is probably based on cross-reacting T cells. This kind of T-cell cross-reactivity does not protect against the infection itself but it prevents severe illness and complications. One application of this is to provide a vaccine that induces a cell-mediated immune response. Such a T-cell response inducing vaccine usually comprises live attenuated protective viruses to induce effective cross-immunity against risk viruses. The live attenuated virus vaccine is capable of attenuating the diabetogenic effect of a risk virus. Preferably the subject is vaccinated both with a live attenuated protective virus and a vaccine comprising inactivated risk viruses or components thereof or antibodies thereto.

Live attenuated coxsackie B virus CBV3 and/or CBV6 may be used in a vaccine for preventing or treating type 1 diabetes. Preferably the use comprises vaccinating a subject with the live attenuated CBV3 or CBV6 or both, and further with an inactive diabetogenic enterovirus or a component thereof, or an antibody thereto. According to one embodiment, the use comprises first vaccinating the subject with at least one of CBV3 and CBV6, and later vaccinating the subject with an inactivated diabetogenic enterovirus or a component thereof. The diabetogenic i.e. the risk enterovirus is preferably selected from the group consisting of CBV1, CBV2 , and CAV16 or any combination thereof. According to one embodiment, the inactivated diabetogenic enterovirus is in the form of a subunit or a nucleic acid of said virus. In another embodiment a subject is vaccinated with live attenuated CBV3 and/or CBV6, and further antibodies against a diabetogenic virus are administered to the subject.

Attenuated viruses are viruses of which the virulence has been reduced. This may be carried out by different methods including serial passage of the virus in cell cultures, antigenic modification by chemical treatments, construction of recombinant or chimeric viruses, mutagenization of viral genome, deletion or insertion of certain gene regions, selection of temperature sensitive mutants or irradiation. Alternatively, the enteroviruses may be attenuated natural virus isolates or infectious virus cDNA or RNA having reduced capability to cause clinical disease.

The live attenuated enterovirus is conveniently formulated into a mucosal vaccine composition, which may be given perorally, sublingually, intranasally, as inhalation, or per rectum. Usually it is administered orally. Each immunizing dose includes infective viruses or infective RNA or cDNA in a titer, which is able to produce infection or activation of the innate or adaptive immune system or induce regulatory T-cells or regulatory cytokines in humans. This dose would correspond to that which is used in the traditional Sabin-type live oral poliovirus vaccine including a minimum of 10^{5.5}- 10⁶ TCID₅₀ for poliovirus Type 1, 10⁵ TCID₅₀ for poliovirus type 2 and 10^{5.5} - 10^{5.8} TCID₅₀ for poliovirus type 3 live attenuated Sabin strains of polioviruses. The dose may also be another, if it has been confirmed to be safe and infectious or able to activate the innate or adaptive immune system. (TCID = tissue culture infectious dose; TCID₅₀= the dose which infects 50% of the cultures.)

The diabetogenic enterovirus, which may be administered in addition to the live CBV3 or CBV6, is inactivated, or the vaccine contains only a component of the diabetogenic virus. The "component" may be an immunogenic structure of the virus such as a subunit thereof, or a nucleic acid fragment such as part of the genome of the virus. The component may also be synthetically produced or modified. Thus the vaccine may include whole viruses, the infectivity of which has been inactivated, or subunit vaccines containing certain antigenic structures, proteins or peptides of the virus, or their combination (such as virus like particles), or fragments of viral RNA or cDNA encoding the whole virus or individual viral proteins or inactivated forms of the virus. Inactivated vaccines may be produced by propagating the virus in cell cultures and by purifying it from infected cells and culture media by high-speed centrifugation in a density gradient formed by sucrose or other high-density media. Alternatively the virus may be purified by chromatography. The infectivity of the purified viruses is destroyed by inactivating the viruses by chemical treatment (e.g. formalin inactivation like that used to produce inactivated polio virus vaccine), irradiation or heat treatment. Subunit vaccines may consist of purified viral proteins or recombinant viral proteins, synthetic peptides corresponding to viral antigenic epitopes, virus like particles or empty viral capsids, which are produced during infection but lack the viral genome. These subunit vaccines can be administered either as such or conjugated to haptens or carriers (e.g. ISCOM particles, chitosan, TLR agonists, biodegradable microparticles).

Alternatively the vaccine against a diabetogenic virus comprises an antibody against at least one of said enteroviruses. This antibody may be raised against the whole virus or against an immunogenic component thereof, or it may be recombinantly produced. The term antibody as used herein includes fragments of antibodies such as Fab-fragments and scFv fragments.

The vaccines against diabetogenic viruses can be given parenterally by injections, perorally, intradermally, transcutaneously, sublingually, intranasally, as inhalation, or per rectum. Each immunizing dose includes viral structures in a titer, which is able to induce proper immune response in humans. This dose would correspond to that used in Salk-type inactivated poliovirus vaccine including 1.8 - 2 µg of viral protein per each dose and 20 - 40 antigenic D-units of poliovirus type 1, 4 - 8 antigenic D-units of poliovirus type 2 and 16-32 antigenic D-units of poliovirus type 3. The dose may also be another, if it has been confirmed to be safe and immunogenic or able to stimulate the immune system.

The vaccines disclosed herein are formulated into pharmaceutical compositions comprising a "pharmaceutically effective amount" of the attenuated virus, or of the inactivated virus, its component, or antibody. A "pharmaceutically effective amount" is an amount, which is able to elicit an immune response that is able to induce immunity against the virus, either by eliciting neutralizing antibodies or a cell-mediated response, or both. In case of antibodies a pharmaceutical effective amount is on that mediates a protective immune response against the virus.

In addition to the active ingredients that elicit immune stimulation the pharmaceutical composition may comprise pharmaceutically acceptable excipients, carriers, haptens and adjuvants. Excipients, carriers, haptens and adjuvants may include for example phenoxyethanol, magnesium chloride, sucrose, thiomersal, formaldehyde, phenol, antibiotics (preservatives) or aluminium salts, polymer microparticles, ISCOM particles, carrier proteins (e.g. cholera toxin), liposomes, protein micelles (haptens/adjuvants), or TLR agonists or other activators of the innate immune system.

The protective enterovirus vaccines and the risk-enterovirus vaccines may be given serially or simultaneously in any order and in any combinations. According to one embodiment the vaccine comprising the protective serotype(s) is given first, and the risk enterovirus vaccine later. In one preferred embodiment the subject is vaccinated with a combination of live attenuated CBV3 and CBV6, and further with an inactivated virus vaccine comprising at least CBV1 or a component thereof. The inactivated vaccine may be a cocktail of serotypes including at least CBV1, CBV2 and CAV16.

In a preferred embodiment of the invention the vaccine against the risk/diabetogenic virus does not contain neither serotype CBV3 nor CBV6, or their components or antibodies. These serotypes should be excluded from said vaccine so as to avoid elimination of the protective serotypes by the vaccine in the recipient

The live attenuated vaccine comprising CBV3 and/or CBV6 is preferably administered to a child within 5 years after birth, and more preferably within 3 years, especially within 2 years, and most preferably within 1 year after birth, with boosters given later in life to prevent or treat type 1 diabetes. It can for example be given at the age or 3, 6 or 12 months, with boosters at older age.

The diabetogenic effect of a risk virus such as CBV1 can be attenuated by exposure to the non-diabetogenic CBV3 or CBV6, which primes the immune system to mount an efficient immune response during exposure to CBV1. Usually, the live vaccine with protective serotypes is given first, and the inactivated vaccine with risk serotypes later. Still, the vaccines may also be given in the opposite order or simultaneously.

The inactivated vaccine comprising at least one risk serotype may also be administered by the regime suggested above. The inactivated vaccine can also be given to pregnant mothers to prevent type-1-diabetes in the baby, or prenatally to the pregnant mother and postnatally to the baby. Vaccine induced antibody response of the mother protects the child because IgG class maternal antibodies are transferred to the fetus through the placenta and are thus protecting the child until the age of 6-12 months when maternal antibodies disappear from the child's circulation.

The vaccination regime can be used in the whole population or in subpopulations carrying increased risk for type 1 diabetes. Such high-risk groups may include mothers or children with HLA-conferred disease susceptibility to type 1 diabetes, especially carriers of the HLA DR3 and/or DR4 alleles, subjects with type 1 diabetes in first or second-degree relatives or children testing positive for two or more diabetes-associated autoantibodies.

The vaccines described may be used in preventing and treating type 1 diabetes, in inducing an immune response against a diabetogenic enterovirus, and in eliminating the diabetogenic effect of an enterovirus in a subject in need thereof by vaccinating the subject with live attenuated CBV3 or CBV6 or both.

### Example

### Seroneutralization analyses

Neutralizing antibodies were analyzed against a wide panel of different enterovirus serotypes in the same serum sample which was the first autoantibody positive sample taken during the prospective follow-up. Thus, this time point represents the initiation of the beta-cell damaging process. In addition, antibodies against those serotypes which were found to be interesting in this initial screening were measured at additional time-points to study the timing of infections and their relationship with the initiation of the beta-cell damaging process.

Altogether, seroneutralization analyses were performed using 42 viruses and 522 serum/plasma samples (174 triplets, two control children for each case child). The completeness of the analyses in this virus set varies from 100% to 84% (Echo30) being for a majority of the viruses (35/40) more than 97.7%. In order to study the effect of the strain variation, two serotypes (CBV4 and Echo3) have been analyzed using both the freshly isolated wild type strains (wt) and corresponding reference (ATCC) strains (rs).

The viruses were analyzed in seroneutralization analyses using autoantibody seroconversion date samples. The viruses were CAV4, CAV5, CAV10, CAV16, EV71, CAV9, CBV1, CBV2, CBV3, CBV4-wt, CBV4-rs, CBV5, CBV6, Echo1, Echo2, Echo3-wt, Echo3-rs, Echo4, Echo5, Echo6, Echo7, Echo9, Echo11, Echo12, Echo13, Echo14, Echo15, Echo17, Echo18, Echo19, Echo20, Echo21, Echo25, Echo26, Echo27, Echo29, Echo30, Echo32, Echo33, EV74, EV78 and EV94.

The seroneutralization analyses were carried out using a plaque neutralization assay. In this analysis the ability of the serum/plasma to inhibit a certain virus's ability to form plaques in cell layers was determined as compared to controls, in which fetal calf serum has been used instead of human serum. In the analysis the inhibition has been considered to be significant (positive result, ++) when it has been more than 85%. The inhibition range between 85-75% has been considered as weaker positive (+) and inhibition of less than 75% has been judged as a negative result. Because these analyses have been performed using two different serum/plasma dilutions (1/4 and 1/16) the results have been combined using the classification shown below.

| **Class** | **1:4** | **1:16** |
|---|---|---|
| Max positive (0) | ++ | ++ |
| Highly positive (1) | ++ | + |
| Moderately positive (2) | ++ | - |
| Positive (3) | + | - |
| Negative (4) | - | - |

### Seroneutralization results

The raw data was exported to Stata package and analyzed using conditional logistic regression models to evaluate the risk of certain serotypes to cause T1 D. The results of the conditional logistic regression analysis are given as odds ratios (ORs). If the OR in certain analysis is higher than 1 and the lower limit of the confidence interval (Cl at the level of 95%) remains above 1 the virus can be considered as conferring risk for T1 D. On the other hand, if both the OR and the higher limit of the 95% Cl are below 1, such a serotype can be considered as protective against T1 D. In such cases where OR is either over or below 1 and also the 95% Cl includes values on both side of 1, the result is not statistically significant (P>0.05). In Table 1 the OR and Cl values for the most interesting viruses are presented.

**Table 1. The OR and Cl values of the most interesting serotypes. The statistically significant results are bolded**

| Virus | [OR (Cl)] | [OR (Cl)] | [OR (Cl)] | |
|---|---|---|---|---|
| | 0-3 vs. 4 | 0-1 vs. 2-4 | 0-2 vs. 4 | 0-1 vs. 4 |
| CBV1 | **1.50 (1.02-2.23)** | 1.10 (0.65-1.87) | 1.56 (0.94-2.58) | 1.39 (0.68-2.85) |
| CBV3 | **0.39 (0.18-0.82)** | 0.56 (0.24-1.34) | **0.36 (0.15-0.85)** | 0.50 (021-1.20) |
| CBV6 | **0.64 (0.41-0.97)** | 0.57 (0.21-1.59) | 0.86 (050-1.51) | 049 (0.16-1.51) |

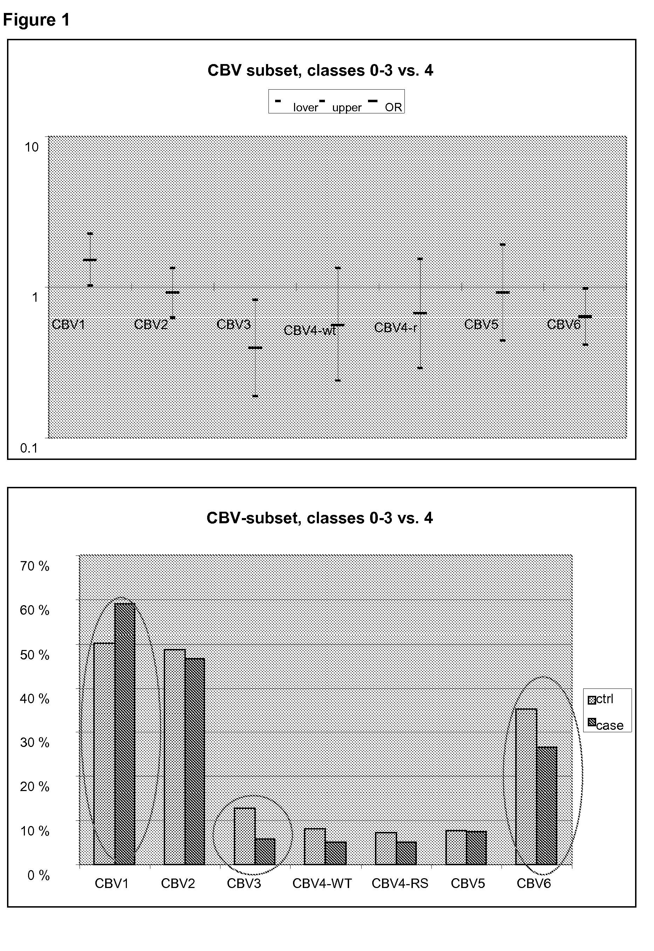

The prevalence of antibodies (percentage of children having neutralizing antibodies against each virus serotype) was also calculated. Figure 1 shows the results of the CBV-subset. The data represent the whole cohort. Classes 0-3 vs. 4, ORs are shown in the upper panel, and seroprevalence in the lower panel. Statistically significant results are marked with circles.

It was found that CBV3 and CBV6 are protective serotypes, while CBV1 is a clear risk serotype for type 1 diabetes. These associations were first drawn from seroneutralization results of a single cross-sectional time-point representing the first autoantibody positive sample, and were later confirmed in multi-time point analyses in which the timing of infections was analyzed in more detail. In these multi-time-point analyses the clearest result was the strong association of the CBV1 risk effect to the six month time window immediately preceding the first detection of type 1 diabetes related autoantibodies. Other CBV serotypes than CBV1, CBV3 and CBV6 did not show such a clear risk or protective effect in the single time-point analysis carried out at autoantibody seroconversion.

### Multi-time point seroneutralization analysis with CBV1, CBV3 and CBV6

To clarify the possible order and timing of CBV1, CBV3 and CBV6 infections, the following time points were selected for the seroneutralization analyses:
- 12 months before autoantibody seroconversion
- 6 months before autoantibody seroconversion
- point (first detection of autoantibodies; these samples were analyzed previously)
- 12 months after autoantibody seroconversion
- time of the diagnosis of type 1 diabetes

According to this plan approximately 1250 new samples fulfilling the criteria above were identified, collected, anonymized and tested.

All samples were screened using 1/4 and 1/16 serum dilutions. In this report the class comparison 03- vs. 4 and sensitivity analyses 1 and 2 has been generally utilized, except in the tight criteria analyses (explained below) in which infections were diagnosed by subjective judgments done by two independent researchers on the basis of pre-fixed criteria listed below for acute infection:
The acute infection was diagnosed according to following criteria which had to be true for a classified infection:
   - A seroconversion from titer 0 to titer 4 or higher
   - The titer is 16 in at least one of the following samples
   - All follow-up samples remain positive

All analyses were done blindly without knowing the case-control status of the child.

### Temporarily peaking antibodies and maternal antibodies

The accumulated data from new time points concerning CBV1, CBV3 and CBV6 clearly showed that the previously analyzed 1/4 and 1/16 dilutions at the time of seroconversion to autoantibody positivity do not provide the whole picture of the infection histories of all children. When these new time points data are taken into account, transient increases in antibody titers were seen against all of these three serotypes either separately or in different combinations in many children. These transient rises may have different explanations: In some cases they are clearly remnants of maternal antibodies. In other cases the reason may be antibody cross-reactivity between closely related serotypes. This issue has been studied more thoroughly in a separate sub-study. In addition, it is possible that the virus strain used in the neutralizing assay is antigenically different from the one causing the infection, and then it may not detect low antibody titers. Furthermore, we cannot rule out the unlikely possibility that some samples may also contain other substances than antibodies that may inhibit virus growth. Finally, there is always a possibility of variation in the assay performance due to technical reasons.

How to deal with this quandary? We have approached this concern using a three-sided strategy:
1. minimizing the influence of wrong negatives = loose criteria
2. minimizing the influence of wrong positives = tight criteria
3. removing the effect of maternal antibodies, otherwise like loose criteria = middle criteria

The loose criteria approach means that basically all results from different time points have been accepted and analyzed as they are. In the middle criteria approach all other results have been accepted except those that have possibly been biased by maternal antibodies. In the tight criteria approach we have carefully gone through the data and excerpted only those infections which fulfill previously set criteria of acute infection.

### Multi-time point statistical analyses

All statistical analyses presented are based on conditional logistic regression analyses. Three types of analyses were carried out for this data set. 1) The timing of infections was determined and the time-relationship between infections and appearance of autoantibodies was analyzed. In other words, the frequency of infections among case children were compared to that in control children in each time window separately. 2) The mutual order of CBV1, CBV3 and CBV6 infections was analyzed separately in case and control children and the interactions between the three serotypes were also analyzed. 3) The new time point results were analyzed similarly as described above by comparing cases and controls in each time point separately. These analyses were carried out in the whole group and in different subgroups according to the following list:
- Total data set from the whole cohort
- Gender
- Age
- HLA genotype
- Residence area
- Combinations of different AABs in certain follow-up samples
- Diagnosis of diabetes
- Cumulative protective effect

Seroneutralization analyses of these three viruses have been carried out using a plaque neutralization assay as described above.

Statistical analyses in loose and middle criteria approaches were done according to combined classes 0-3 vs. 4 and also sensitivity analyses, in which class 3 (sensitivity analysis 1) or both classes 2 and 3 (sensitivity analysis 2) are removed from the data set, were utilized. In the tight criteria approach the sensitivity analyses were not done due to its intrinsic "sensitivity" character.

### Timing of the infections

The timing of infections caused by the three CBV serotypes of interest was approached in the following way: First, the recognized infections were categorized to different time windows relative to the date of seroconversion to autoantibody positivity (AAB+ date). The used windows were as follows:
0. No infections or infection after the AAB+ date
1. Infection more than 12 months before the AAB+ date
2. Infection between 12 and 6 months before the AAB+ date
3. Infection within 6 months before the AAB+ date

In addition to using these time-windows separately, analyses were also performed by combining some of these windows.

In the loose criteria approach the first positive result was accepted as an infection regardless of the possibility of maternal antibodies or the possibility that results of the later time point samples turn to negative. The middle criteria approach was done similarly, except that those results that were biased by the identified maternal antibodies were nullified. This judgement was done by two independent experts who evaluated the data carefully and discarded those results in which the maternal antibodies could be the cause of the positivity. In the tight criteria approach the similar judgement procedure were applied. In short the acute infection according to tight criteria were as follows:
- a seroconversion from titer 0 to titer 4 or higher
- the titer is 16 in at least one of the following samples
- all follow-up samples remain positive

In addition to using these time-windows separately, analyses were also performed by combining some of these windows together.

The timing of infections caused by the CBV1 risk serotype in these time windows is summarized in Tables 2 and 3. These analyses were done using the loose criteria and middle criteria and the risk effect of infection was analyzed by comparing its frequency in different time-windows to that in window zero (see above). The basic statistical analyses i.e. classes 0-3 vs. 4. and sensitivity analyses 1 and 2 were done.

**Table 2. CBV1 timing conditional logistic regression analysis, other windows vs. window 0. Loose criteria approach with classes 0-3 vs. 4 and sensitivity analyses 1 and 2.**

| **CBV1** | **Odds ratio** | **P-value** | **95% Conf. Interval** | |
|---|---|---|---|---|
| **Class 0-3 vs. 4** | | | | |
| Over 12 months before AAB+ date | 1.77 | 0.064 | 0.97 | 3.25 |
| 12-6 months before AAB+ date | **2.24** | **0.007** | **1.25** | **4.00** |
| 6-0 months before AAB+ date | **3.56** | **< 0.001** | **1.92** | **6.62** |
| **Sensitivity 1, Class 0-2 vs. 4** | | | | |
| Over 12 months before AAB+ date | 0.89 | 0.724 | 0.46 | 1.72 |
| 12-6 months before AAB+ date | 1.25 | 0.468 | 0.68 | 2.31 |
| 6-0 months before AAB+ date | **2.19** | **0.018** | **1.15** | **4.17** |
| **Sensitivity 2, Class 0-1 vs. 4** | | | | |
| Over 12 months before AAB+ date | 1.19 | 0.684 | 0.51 | 2.79 |
| 12-6 months before AAB+ date | 0.98 | 0.950 | 0.47 | 2.03 |
| 6-0 months before AAB+ date | 2.09 | 0.067 | 0.95 | 4.59 |

**Table 3. CBV1 timing conditional logistic regression analysis, other windows vs. window 0. Middle criteria approach with classes 0-3 vs. 4 and sensitivity analyses 1 and 2.**

| **CBV1** | **Odds ratio** | **P-value** | **95% Conf. Interval** | |
|---|---|---|---|---|
| **Class 0-3 vs. 4** | | | | |
| Over 12 months before AAB+ date | 1.62 | 0.262 | 0.70 | 3.75 |
| 12-6 months before AAB+ date | 0.77 | 0.417 | 0.41 | 1.44 |
| 6-0 months before AAB+ date | **2.09** | **0.002** | **1.32** | **3.33** |
| **Sensitivity 1, Class 0-2 vs. 4** | | | | |
| Over 12 months before AAB+ date | 1.14 | 0.795 | 0.41 | 3.17 |
| 12-6 months before AAB+ date | 0.66 | 0.307 | 0.30 | 1.46 |
| 6-0 months before AAB+ date | **2.10** | **0.007** | **1.23** | **3.57** |
| **Sensitivity 2, Class 0-1 vs. 4** | | | | |
| Over 12 months before AAB+ date | 0.70 | 0.629 | 0.16 | 3.02 |
| 12-6 months before AAB+ date | 0.36 | 0.078 | 0.11 | 1.12 |
| 6-0 months before AAB+ date | 1.44 | 0.342 | 0.68 | 3.05 |

Interestingly, in these analyses the CBV1 infections are clearly found more often in case children according to the risk hypothesis with infections occurring close to the time of autoantibody seroconversion (at the time window 6-0 months before the AAB+ date). The result is the same in all analyses, except that in sensitivity analysis 2 the statistical significance is missed. Because in this analysis we have accepted all positive antibody results (probably including false positive findings), except maternal antibodies in middle criteria analyses, it is essential to compare these results to those obtained using tight criteria for infections (Table 4).

**Table 4. CBV1 timing conditional logistic regression analysis, other windows vs. window 0. Tight criteria approach.**

| **CBV1** | **Odds ratio** | **P-value** | **95% Conf. Interval** | |
|---|---|---|---|---|
| Over 12 months before AAB+ date | 0.67 | 0.455 | 0.24 | 1.89 |
| 12-6 months before AAB+ date | 1.38 | 0.491 | 0.55 | 3.46 |
| 6-0 months before AAB+ date | **3.76** | **0.001** | **1.68** | **8.44** |

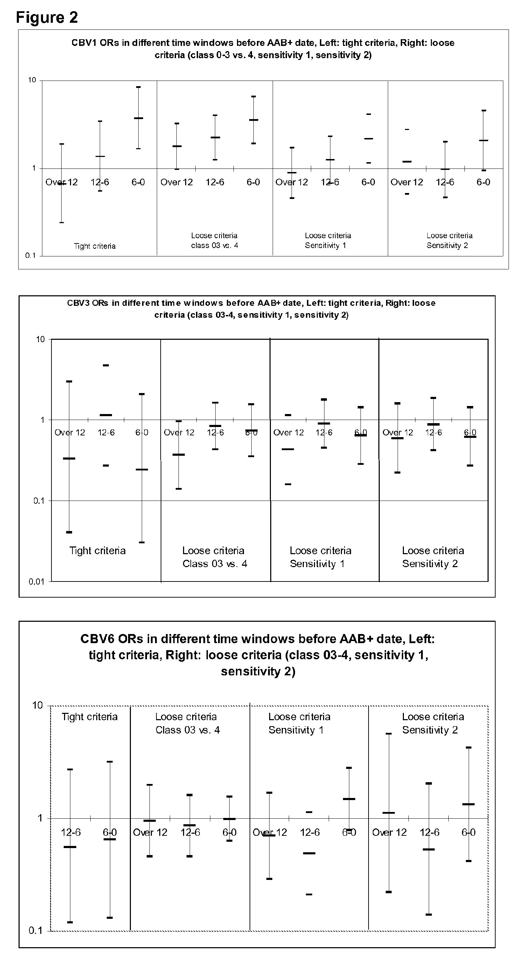

In this analysis, a lot of data have been removed to exclude all possible wrong positives. The results show that a credible dose response curve is observed highlighting the importance of the time window 6-0 months before the AAB+ date and proving a definite risk effect of CBV1 with convincing statistical outcome. Most importantly, because the same 6-0 months before the AAB+ date window was observed to be statistically significant in both the loose, middle and tight criteria approaches, these results cement this time period as a critical one for the CBV1 infection as a risk to induce T1 D. The tight criteria and loose criteria results are presented together in Figure 2, which shows CBV1 ORs in different time windows before AAB+ date, Left: tight criteria, Right: loose criteria (class 0-3 vs. 4, sensitivity 1, sensitivity 2). The dose response curves can be seen with both approaches being the most conspicuous in the tight criteria and loose criteria sensitivity 1 analyses.

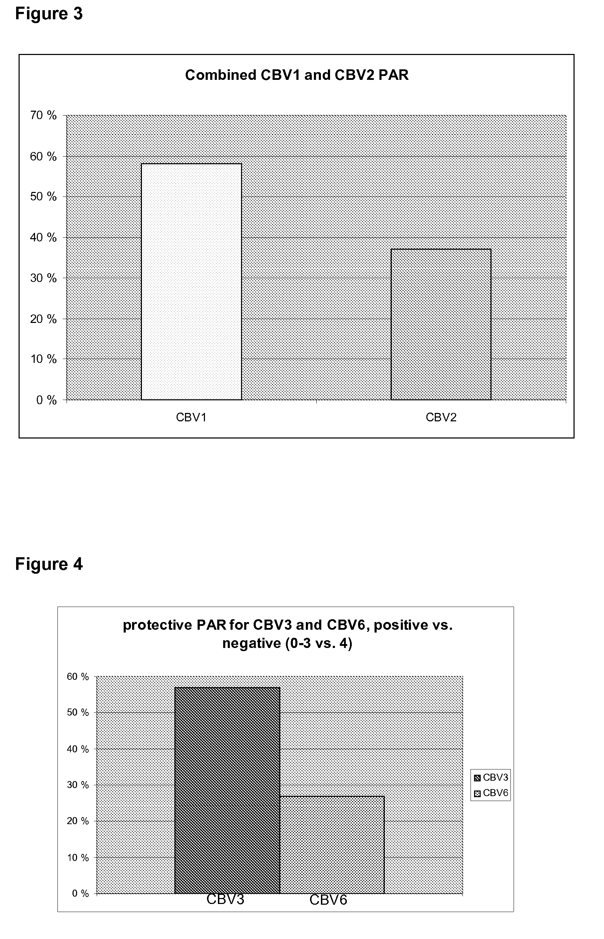

In order to understand the significance of this result let us interpret it in the terms of the population attributable risk (PAR) which estimates the proportion of type 1 diabetes cases which could be prevented by the CBV1 vaccine in the population. Assuming that the OR is 3.76 (= tight criteria result for window 6-0 months before the AAB+ date) and the prevalence of the CBV1 infection is 50% as indicated by the prevalence of CBV1 antibodies in AAB+ time point in control subjects, the PAR equation results in 58% for CBV1 alone (Figure 3). Figure 3 shows the population attributable risk (PAR) for CBV1 calculated using an OR of 3.76 and a prevalence (Pc) of 50% representing the seroprevalence at the time of autoantibody seroconversion in control subjects.

In the following analysis the time windows 6-0 and 12-6 were combined. The risk effect of CBV1 was significant also in this combined time window analysis (Table 5).

**Table 5. CBV1 timing conditional logistic regression analysis, 12-0 window against window 0. Tight criteria approach.**

| **CBV1** | **Odds ratio** | **P-value** | **95% Conf. Interval** | |
|---|---|---|---|---|
| Over 12 months before AAB+ date | 0.66 | 0.434 | 0.24 | 1.85 |
| 12-0 months before AAB+ date | **2.46** | **0.004** | **1.33** | **4.53** |

Because the strongest risk effect was seen in the 6-0 window, this time period was analyzed using another type of comparison. Instead of comparing it against window 0 (which was done in the previous analyses), this window was compared to all other windows in combination. Again, the previous findings showing the critical importance of this time-window were supported by this analysis (Table 6).

**Table 6. CBV1 timing conditional logistic regression analysis, 6-0 window against combined other time windows. Tight criteria approach.**

| **CBV1** | **Odds ratio** | **P-value** | **95% Conf.Interval** | |
|---|---|---|---|---|
| 6-0 months before AAB+ date | **3.78** | **0.001** | **1.69** | **8.43** |

Similar analyses were performed for the protective serotypes CBV3 and CBV6. Tables 7, 8, 9 and 10 present the results of different time points for CBV3 and CBV6, respectively, when analyzed using loose and middle criteria. The tight criteria and loose criteria results are presented in Figure 2 for both CBV3 and CBV6. Protection by CBV3 was seen when infection occurred early. The effect was weakened when maternal antibodies were excluded (middle criteria) suggesting that maternal antibodies may provide part of this protection.

**Table 7. CBV3 timing conditional logistic regression analysis, other windows against window 0. Loose criteria approach.**

| **CBV3** | **Odds ratio** | **P-value** | **95% Conf. Interval** | |
|---|---|---|---|---|
| **Class 0-3 vs. 4** | | | | |
| Over 12 months before AAB+ date | **0.37** | **0.038** | **0.14** | **0.95** |
| 12-6 months before AAB+ date | 0.84 | 0.618 | 0.43 | 1.64 |
| 6-0 months before AAB+ date | 0.74 | 0.439 | 0.35 | 1.57 |
| **Sensitivity 1** | | | | |
| Over 12 months before AAB+ date | 0.43 | 0.086 | 0.16 | 1.13 |
| 12-6 months before AAB+ date | 0.90 | 0.754 | 0.45 | 1.77 |
| 6-0 months before AAB+ date | 0.64 | 0.280 | 0.28 | 1.44 |
| **Sensitivity 2** | | | | |
| Over 12 months before AAB+ date | 0.59 | 0.291 | 0.22 | 1.58 |
| 12-6 months before AAB+ date | 0.87 | 0.724 | 0.42 | 1.84 |
| 6-0 months before AAB+ date | 0.62 | 0.266 | 0.27 | 1.44 |

**Table 8. CBV6 timing conditional logistic regression analysis, other windows against window 0. Loose criteria approach.**

| **CBV6** | **Odds ratio** | **P-value** | **95% Conf. Interval** | |
|---|---|---|---|---|
| **Class 0-3 vs. 4** | | | | |
| Over 12 months before AAB+ date | 0.92 | 0.772 | 0.53 | 1.61 |
| 12-6 months before AAB+ date | 1.53 | 0.098 | 0.92 | 2.52 |
| 6-0 months before AAB+ date | 1.33 | 0.264 | 0.80 | 2.22 |
| **Sensitivity 1** | | | | |
| Over 12 months before AAB+ date | 0.37 | 0.060 | 0.13 | 1.04 |
| 12-6 months before AAB+ date | 0.74 | 0.410 | 0.37 | 1.51 |
| 6-0 months before AAB+ date | 1.04 | 0.912 | 0.52 | 2.08 |
| **Sensitivity 2** | | | | |
| Over 12 months before AAB+ date | 0.26 | 0.227 | 0.03 | 2.33 |
| 12-6 months before AAB+ date | 0.57 | 0.354 | 0.17 | 1.88 |
| 6-0 months before AAB+ date | 0.90 | 0.888 | 0.20 | 3.98 |

**Table 9. CBV3 timing conditional logistic regression analysis, other windows against window 0. Middle criteria approach.**

| **CBV3** | **Odds ratio** | **P-value** | **95% Conf. Interval** | |
|---|---|---|---|---|
| **Class 0-3 vs. 4** | | | | |
| Over 12 months before AAB+ date | 0.50 | 0.535 | 0.06 | 4.47 |
| 12-6 months before AAB+ date | 0.61 | 0.557 | 0.11 | 3.22 |
| 6-0 months before AAB+ date | 0.62 | 0.327 | 0.24 | 1.60 |
| **Sensitivity 1** | | | | |
| Over 12 months before AAB+ date | 0.50 | 0.535 | 0.06 | 4.47 |
| 12-6 months before AAB+ date | 0.61 | 0.557 | 0.11 | 3.22 |
| 6-0 months before AAB+ date | 0.42 | 0.147 | 0.13 | 1.36 |
| **Sensitivity 2** | | | | |
| Over 12 months before AAB+ date | 0.50 | 0.535 | 0.06 | 4.47 |
| 12-6 months before AAB+ date | 0.39 | 0.405 | 0.04 | 3.59 |
| 6-0 months before AAB+ date | 0.47 | 0.213 | 0.14 | 1.54 |

**Table 10. CBV6 timing conditional logistic regression analysis, other windows against window 0. Middle criteria approach.**

| **CBV6** | **Odds ratio** | **P-value** | **95% Conf. Interval** | |
|---|---|---|---|---|
| **Class 0-3 vs. 4** | | | | |
| Over 12 months before AAB+ date | 0.96 | 0.902 | 0.46 | 1.97 |
| 12-6 months before AAB+ date | 0.87 | 0.653 | 0.46 | 1.62 |
| 6-0 months before AAB+ date | 0.99 | 0.969 | 0.63 | 1.56 |
| **Sensitivity 1** | | | | |
| Over 12 months before AAB+ date | 0.70 | 0.433 | 0.29 | 1.69 |
| 12-6 months before AAB+ date | 0.49 | 0.097 | 0.21 | 1.14 |
| 6-0 months before AAB+ date | 1.48 | 0.222 | 0.79 | 2.79 |
| **Sensitivity 2** | | | | |
| Over 12 months before AAB+ date | 1.12 | 0.891 | 0.22 | 5.62 |
| 12-6 months before AAB+ date | 0.53 | 0.358 | 0.14 | 2.04 |
| 6-0 months before AAB+ date | 1.34 | 0.620 | 0.42 | 4.27 |

Tight criteria results for same viruses are shown in table 11.

**Table 11. CBV3 and CBV6 timing conditional logistic regression analysis, other windows against window 0. Tight criteria approach. *There were no CBV6 infections in cases over 12 months window according to tight criteria approach. Therefore statistical results could not be calculated for that window. However, in controls infections were recognized also in this time window supporting the protective effect of CBV6.**

| **CBV3** | **Odds ratio** | **P-value** | **95% Conf. Interval** | |
|---|---|---|---|---|
| Over 12 months before AAB+ date | 0.33 | 0.326 | 0.04 | 2.95 |
| 12-6 months before AAB+ date | 1.13 | 0.870 | 0.27 | 4.74 |
| 6-0 months before AAB+ date | 0.24 | 0.195 | 0.03 | 2.09 |
| **CBV6** | | | | |
| Over 12 months before AAB+ date | -* | -* | -* | -* |
| 12-6 months before AAB+ date | 0.56 | 0.468 | 0.12 | 2.70 |
| 6-0 months before AAB+ date | 0.65 | 0.592 | 0.13 | 3.21 |

In most of these analyses for CBV3 and CBV6 a trend can be seen that the former the time window is the more protective character both these viruses show. These results as such are logical: The protective virus should precede the risk virus, assuming that the protective effect is mediated by immunological cross-protection. The same trend is seen for both viruses in most of the analyses.

As a conclusion from the timing analyses it can be seen that the critical timing of the risk virus CBV1 infection falls into the time window one year and especially 6 months before the AAB+ date. The results also supported the protective properties of CBV3 and CBV6.

If protective, the lack of said virus can be seen as a risk for T1 D. Therefore the inverse prevalence and inverse OR was used for protective PAR% calculation using positive versus negative (0-3 vs 4). The result of the protective PAR% calculations was 57% for CBV3 and 27% for CBV6, as also shown in Figure 4.

### Order of the infections - chronology

In this analysis different possibilities of the order of infections were first classified to following classes:
0 = not risk or protective viruses at any time point
1 = first infection by the risk virus, CBV1
2 = first infection by the protective virus, CBV3 or CBV6, alone or both
3 = first infection by the risk and protective virus in the same time-window

In this first analysis the classes "first risk infection" (class 1 above) and "combined protective class" (class 2) were analyzed. Statistical comparisons were made against class "not risk or protective viruses at any time-point" (class 0). Results of these analyses are summarized in Table 12 (tight criteria), Table 13 (loose criteria) and Table 14 (middle criteria). In these analyses the follow-up period till the AAB+ date has been taken into account.

**Table 12. Order of the infections (conditional logistic regression analysis), compared to class 0. Tight criteria approach.**

| **Order** | **Odds ratio** | **P-value** | **95% Conf. Interval** | |
|---|---|---|---|---|
| First risk | 1.29 | 0.290 | 0.81 | 2.06 |
| First protective | **0.58** | **0.098** | **0.31** | **1.10** |

**Table 13. Order of the infections (conditional logistic regression analysis), compared to class 0. Loose criteria approach.**

| **Order** | **Odds ratio** | **P-value** | **95% Conf. Interval** | |
|---|---|---|---|---|
| **Class 0-3 vs. 4** | | | | |
| First risk | 1.56 | 0.247 | 0.73 | 3.32 |
| First protective | 0.87 | 0.727 | 0.40 | 1.90 |

**Table 14. Order of the infections (conditional logistic regression analysis), compared to class 0. Middle criteria approach.**

| **Order** | **Odds ratio** | **P-value** | **95% Conf. Interval** | |
|---|---|---|---|---|
| **Class 0-3 vs. 4** | | | | |
| First risk | **3.31** | **<0.001** | **1.83** | **5.99** |
| First protective | **2.10** | **0.025** | **1.10** | **4.00** |

All these three analyses showed trends supporting especially the hypothesis of CBV1's risk character but also the hypothesis of protective viruses. In the second type of analysis the classes 0 and 2 were combined and class 1 was analyzed against this new combined class. In this analysis the risk effect of CBV1 becomes stronger (Table 15).

**Table 15. CBV1 infections first (conditional logistic regression analysis), compared to combined classes 0 and 2. Tight criteria approach.**

| **Order** | **Odds ratio** | **P-value** | **95% Conf. Interval** | |
|---|---|---|---|---|
| First risk | 1.40 | 0.151 | 0.88 | 2.22 |

To study the protective effect more closely the risk class (class 1) was combined with the 0-class and this combined class was compared with class 2. In this analysis the protective effect gets stronger and the P-value comes very close to statistical significance (Table 16).

**Table 16. Protective infections first (conditional logistic regression analysis), compared to combined classes 0 and 1. Tight criteria approach.**

| **Order** | **Odds ratio** | **P-value** | **95% Conf. Interval** | |
|---|---|---|---|---|
| First protective | **0.55** | **0.060** | 0.29 | 1.03 |

In the following analysis the protective viruses CBV3 and CBV6 have been handled as separate cases and the analyses were done using both tight, middle and loose criteria approaches (Tables 17, 18 and 19).

**Table 17. Order of the infections (conditional logistic regression analysis), compared to class 0. CBV3 vs. CBV1 and CBV6 vs. CBV1 handled as separate groups. Tight criteria approach.**

| **CBV3 vs. CBV1** | **Odds ratio** | **P-value** | **95% Conf. Interval** | |
|---|---|---|---|---|
| CBV1 first | 1.25 | 0.338 | 0.79 | 1.95 |
| CBV3 first | 0.41 | 0.104 | 0.14 | 1.20 |
| **CBV6 vs. CBV1** | | | | |
| CBV1 first | 1.38 | 0.170 | 0.87 | 2.17 |
| CBV6 first | 0.62 | 0.236 | 0.29 | 1.36 |

**Table 18. Order of the infections (conditional logistic regression analysis), compared to class 0. CBV3 vs. CBV1 and CBV6 vs. CBV1 handled as separate groups. Middle criteria approach.**

| **Class 0-3 vs. 4** | **Odds ratio** | **P-value** | **95% Conf. Interval** | |
|---|---|---|---|---|
| **CBV3 vs. CBV1** | | | | |
| CBV1 first | **2.76** | **<0.001** | **1.75** | **4.34** |
| CBV3 first | 1.30 | 0.706 | 0.33 | 5.05 |
| **CBV6 vs. CBV1** | | | | |
| CBV1 first | **3.67** | **<0.001** | **2.00** | **6.72** |
| CBV6 first | **2.48** | **0.007** | **1.28** | **4.80** |

**Table 19. Order of the infections (conditional logistic regression analysis), compared to class 0. CBV3 vs. CBV1 and CBV6 vs. CBV1 handled as separate groups. Loose criteria approach.**

| **Class 0-3 vs. 4** | **Odds ratio** | **P-value** | **95% Conf. Interval** | |
|---|---|---|---|---|
| **CBV3 vs. CBV1** | | | | |
| CBV1 first | 1.24 | 0.488 | 0.68 | 2.26 |
| CBV3 first | **0.41** | **0.057** | 0.16 | 1.03 |
| **CBV6 vs. CBV1** | | | | |
| CBV1 first | **2.37** | **0.072** | 0.92 | 6.06 |
| CBV6 first | 1.85 | 0.217 | 0.70 | 4.91 |

According to tight criteria analyses the trends were as expected showing risk trend for CBV1 and protective trends for CBV3 and CBV6. In the middle criteria analysis, once again, the risk character of CBV1 was clearly evidenced. In the loose criteria analyses the protective effect of CBV3 clearly stands out and in CBV6 vs. CBV1 comparison the risk effect of CBV1 is obvious.

In conclusion, the chronology studies provided supportive data for the risk character of CBV1 and the protective character especially for CBV3 and to a lesser extent for CBV6, too. Based on these findings in can be concluded that the chronology of these infections influence the risk effect of CBV1. High risk is associated with CBV1 infection without prior exposure to CBV3 or CBV6. On the other hand, the risk effect is attenuated by previous exposure to CBV3 or CBV6.

### Antibody cross-reactivity within CBV serotypes

### Cross-reactivity analyses in the seroneutralization study cohort

Immunological cross-reactivity between CBV1, CBV3 and CBV6 was studied in the study cohort in the following two sample series:
a) samples from children, in which a titer against one of the interesting viruses (CBV1, 3 or 6) was at least 1/16 and for the other of these viruses it was lower but at a detectable level (classes 1-3) and
b) samples from children in which one of the CBV serotypes was detected in stool sample. In both cases, serum samples from later time points were studied as well, if possible. In all cases antibodies against all CBV serotypes were measured.

There were altogether eight children in whom a CBV serotype was detected in his/her stool sample based on direct sequence analysis (group b). Neutralizing antibodies were analyzed in these children against serotypes CBV1-6. In all the eight children the antibody response was specifically induced with high titers against the serotype detected in stools in the serum sample collected just before or straight after the CBV positive stool sample (titer at least 1:512).

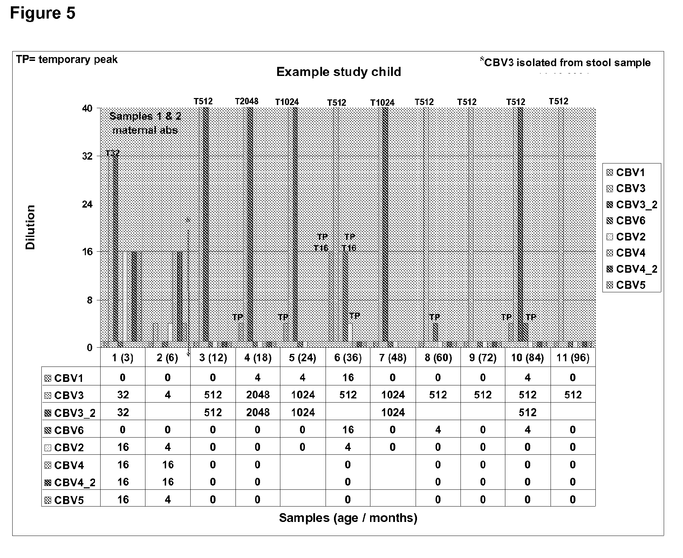

Figure 5 shows an example of one child in whom a CBV3 serotype was detected in the stool sample. GenBank accession numbers and origin for the Coxsackie B viruses used in seroneutralization: CBV1: EU147493; CBV3: AY896763 (isolated from this example study child); CBV3_2: M33854; CBV6: AF039205; CBV2: AM159197; CBV4: X05690; CBV4_2: AF160018; CBV5: DQ092797.

The data table contains titers for each antibody in the sample. If the sample is not analyzed for antibodies against a specific virus, there is a blank point for the virus in question. The numeric end-point titer is shown in the chart above each column containing the prefix T if end-point titration was done for the sample. The first two samples of this child were collected at the age of 3 and 6 months, and contained maternal antibodies. A homotypic antibody response against CBV3 was induced in sample no. 3, which is the following serum sample collected after the CBV3 positive stool sample.

In all the eight children from whom a CBV serotype was detected in the stool sample, one or more low-titer responses against other serotypes than the one detected in stools were also observed. These low-titer responses may represent a true infection by another serotype, but they may also represent cross-reactive responses especially when coinciding with the high-titer response against the serotype detected in stools. Low-titer responses were often transient and seen mainly in samples where homotypic titers were high, but also in samples having no other infections at the same time. In Figure 6 the child has eight low-titer responses (temporary peaks) which were detected after the homotypic response to CBV3.

When children selected for groups a (23 children) and b (8 children) were looked as one group a trend shown in Table 20 was found. Four children having high homotypic responses for CBV3 had 1-3 low-titer temporary peaks (altogether nine temporary peaks) for CBV1 in the samples where CBV3 titer was high. Further, there were four children each having one low-titer temporary peak for CBV2 in the samples where CBV3 titer was high. In addition, seven children had altogether thirteen low-titer temporary peaks for CBV6 in the samples where CBV3 titer was high.

**Table 20. The main trends of low-titer temporary peaks suggesting cross reactivity between different CBV serotypes. CBV3 homotypic responses were found from 18 children (from 65 samples) of which 12 children (in 26 samples) also had low-titer temporary responses in CBV1, -2 or -6.**

| **Homotypic response** | **Low-titer temporary response in** | **Number of samples where low-titer temporary response** | **Number of children having low-titer temporary response** |
|---|---|---|---|
| CBV3 | CBV1 | 9 | 4 |
| | CBV2 | 4 | 4 |
| | CBV6 | 13 | 7 |

In conclusion, according to the cohort analyses CBV3 seems to have cross-reactivity with CBV1, CBV2 and CBV6.

### Monospecific antisera

A panel of monospecific sera was ordered directly from ATCC. The sera were raised in horse against all other CBV viruses (ATCC reference strains), except CBV2. A cross neutralization assay was performed using these monospecific sera and CBV strains available.

According to the assay, the CBV6 antiserum weakly cross-neutralized both CBV1 and CBV3 viruses (Table 21). No reaction vice-versa was seen with CBV1 or CVB3 antisera against CBV6 virus. In addition, CBV5 antiserum weakly cross-reacted with CBV6.

The overall titers were relatively low compared to monospecific antibody set no. 1, especially for CBV1 and CBV3 antisera. The cross-neutralization assay was performed also using the very ATCC CBV reference strains against which the antisera were raised to see if the virus strain has any effect on the serum titers. The cross-neutralization results and serum titers were, nevertheless, identical to those performed with wild-type viruses.

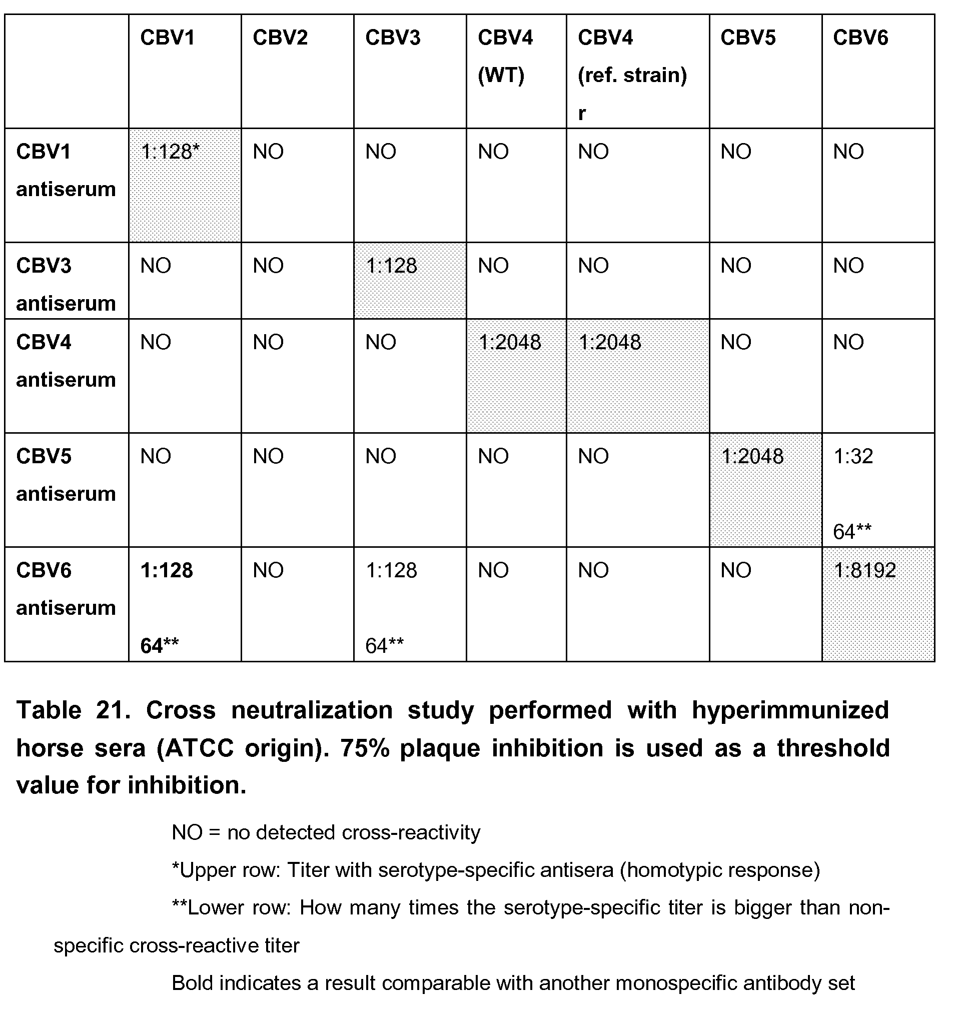

In conclusion, if the illogical CBV4 result is removed, there is one common result, according to which the CBV1 virus weakly cross-reacts non-specifically with CBV6 serum. In addition, there are orphan results indicating weak cross reactivity between CBV3 virus/CBV6 antiserum, CBV5 virus/CBV1 & CBV3 antisera and CBV6 virus/CBV1 & CBV5 antisera.

### Conclusions

When all analyses are combined, we can conclude that the statistically significant association between enteroviruses and disease process leading to type 1 diabetes was confirmed and strengthened. Enteroviruses can have a risk effect (i.e. induce the diabetic disease process) or a protective effect. Most importantly these studies prove that it is feasible to develop an enterovirus-based vaccine against type 1 diabetes, which vaccine comprises at least the main diabetogenic enterovirus serotype CBV1.

The most interesting results are related to the CBV serotypes. In this subgroup the strongest single discovery was the definite risk character of CBV1. It was first identified in relation to the time point of seroconversion to autoantibody positivity by seroneutralization analyses showing that CBV1 infections precede the initial autoantibody seroconversion. Later this result was confirmed by proving that the CBV1 infections hit more often the case children as compared to controls in the time window which immediately precedes autoantibody seroconversion. On the other hand, strong protective properties were associated with CBV serotypes CBV3 and CBV6. Also these associations were first identified by seroneutralization analyses in relation to the time of seroconversion to autoantibody positivity. The timing and chronology studies supported specific interactions between CBV1, CBV3 and CBV6 where the diabetogenic effect of CBV1 is attenuated by prior exposure to either CBV3 or CBV6 or both. This fits well with immunological cross-protection between these antigenically related serotypes. In the antibody-based cross-reactivity analyses immunological cross-reactivity was indeed observed between CBV serotypes and CBV1, CBV3 and CBV6 were found to be involved in many of them.

### Multi-time point seroneutralization analysis further including CBV2, CBV4 and CBV5

In this analysis also the remaining three CBV serotypes were analyzed in similar multi-time-point setting as CBV1, CBV3 and CBV6.

### Timing of infections

To clarify the possible timing of CBV subgroup infections, the same time points were selected for the seroneutralization analyses as described above.

The results presented below are based on the seroneutralization analysis of about 2000 samples for each of these CBV viruses. In order to study the timing of infections caused by the six CBV serotypes the recognized infections were categorized to different time windows relative to the date of seroconversion to autoantibody positivity (AAB+ date). The used windows were numbered from 0 to 3 as described above.

The same loose, middle and tight criteria approaches were used as described above. Statistical analyses were done using conditional logistic regression. The results of statistical analyses for the six CBV serotypes are shown in Tables 22-27.

**Table 22. CBV1 infection timing analysis. Loose criteria approach with classes 0-3 vs. 4.**

| **CBV1** | **Odds ratio** | **P-value** | **95% Conf.Interval** | |
|---|---|---|---|---|
| **Class 0-3 vs. 4** | | | | |
| Over 12 months before AAB+ date | **1.89** | **0.035** | **1.05** | **3.40** |
| 12-6 months before AAB+ date | **2.27** | **0.005** | **1.28** | **4.03** |
| 6-0 months before AAB+ date | **3.54** | **<0.001** | **1.95** | **6.42** |

**Table 23 CBV2 infection timing analysis. Loose, Middle and Tight criteria approaches with classes 0-3 vs. 4.**

| **CBV2** | **Odds ratio** | **P-value** | **95% Conf. Interval** | |
|---|---|---|---|---|
| **Loose criteria** | | | | |
| Over 12 months before AAB+ date | 0.79 | 0.441 | 0.44 | 1.43 |
| 12-6 months before AAB+ date | 1.38 | 0.236 | 0.81 | 2.34 |
| 6-0 months before AAB+ date | **2.32** | **0.001** | **1.40** | **3.84** |
| **Middle criteria** | | | | |
| Over 12 months before AAB+ date | 0.80 | 0.523 | 0.41 | 1.58 |
| 12-6 months before AAB+ date | 1.02 | 0.946 | 0.54 | 1.93 |
| 6-0 months before AAB+ date | **2.29** | **0.001** | **1.43** | **3.66** |
| **Tight Criteria** | | | | |
| Over 12 months before AAB+ date | 0.88 | 0.838 | 0.26 | 3.00 |
| 12-6 months before AAB+ date | 1.00 | 0.994 | 0.33 | 3.03 |
| 6-0 months before AAB+ date | **2.44** | **0.059** | **0.97** | **6.15** |

**Table 24 CBV3 infection timing analysis. Loose criteria approach with classes 0-3 vs. 4.**

| **CBV3** | **Odds ratio** | **P-value** | **95% Conf.Interval** | |
|---|---|---|---|---|
| **Class 0-3 vs. 4** | | | | |
| Over 12 months before AAB+ date | 0.42 | 0.058 | 0.18 | 1.03 |
| 12-6 months before AAB+ date | 0.84 | 0.609 | 0.43 | 1.63 |
| 6-0 months before AAB+ date | 0.69 | 0.331 | 0.33 | 1.45 |

**Table 25 CBV4 infection timing analysis. Loose, Middle and Tight criteria approaches with classes 0-3 vs. 4.**

| **CBV4** | **Odds ratio** | **P-value** | **95% Conf.Interval** | |
|---|---|---|---|---|
| **Loose criteria** | | | | |
| Over 12 months before AAB+ date | 0.51 | 0.135 | 0.21 | 1.24 |
| 12-6 months before AAB+ date | 1.05 | 0.887 | 0.53 | 2.09 |
| 6-0 months before AAB+ date | 1.07 | 0.868 | 0.48 | 2.41 |
| **Middle criteria** | | | | |
| Over 12 months before AAB+ date | 0.50 | 0.380 | 0.11 | 2.35 |
| 12-6 months before AAB+ date | -* | -* | -* | -* |
| 6-0 months before AAB+ date | 0.45 | 0.363 | 0.08 | 2.50 |
| **Tight Criteria** | | | | |
| Over 12 months before AAB+ date | 1.00 | 0.999 | 0.09 | 11.04 |
| 12-6 months before AAB+ date | -* | -* | -* | -* |
| 6-0 months before AAB+ date | 0.59 | 0.685 | 0.05 | 7.43 |

*Due to the low overall frequency of CBV4 infections there were no CBV4 infections in cases in 12-6 months window according to middle and tight criteria approaches.

**Table 26. CBV5 infection timing analysis. Loose, Middle and Tight criteria approaches with classes 0-3 vs. 4.**

| **CBV5** | **Odds ratio** | **P-value** | **95% Conf.Interval** | |
|---|---|---|---|---|
| **Loose criteria** | | | | |
| Over 12 months before AAB+ date | 0.56 | 0.209 | 0.23 | 1.38 |
| 12-6 months before AAB+ date | 1.11 | 0.755 | 0.57 | 2.19 |
| 6-0 months before AAB+ date | 1.60 | 0.211 | 0.76 | 3.37 |
| **Middle criteria** | | | | |
| Over 12 months before AAB+ date | -* | -* | -* | -* |
| 12-6 months before AAB+ date | 0.88 | 0.849 | 0.23 | 3.35 |
| 6-0 months before AAB+ date | 1.45 | 0.488 | 0.51 | 4.14 |
| **Tight Criteria** | | | | |
| Over 12 months before AAB+ date | -§ | -§ | -§ | -§ |
| 12-6 months before AAB+ date | -§ | -§ | -§ | -§ |
| 6-0 months before AAB+ date | -§ | -§ | -§ | -§ |

*There were no CBV5 infections in cases in over 12 months window according to middle criteria approach.
§ ln tight criteria approach the number of the observations decreased so low that no statistically analyzed results could be generated.

**Table 27. CBV6 infection timing analysis. Loose criteria approach with classes 0-3 vs.**

| **CBV6** | **Odds ratio** | **P-value** | **95% Conf.Interval** | |
|---|---|---|---|---|
| **Class 0-3 vs. 4** | | | | |
| Over 12 months before AAB+ date | 0.96 | 0.874 | 0.56 | 1.65 |
| 12-6 months before AAB+ date | 1.54 | 0.090 | 0.94 | 2.54 |
| 6-0 months before AAB+ date | 1.35 | 0.234 | 0.82 | 2.22 |

### Conclusions

In addition to CBV1 also the closely related CBV2 was found to be a risk virus in multi-time-point analyses especially in the 6-0 months before AAB+ time window. Thus, the multi-time-point analyses produced a good second candidate for the preventive vaccine cocktail without forgetting the protective viruses found earlier.

PAR was also calculated for CBV2 using loose criteria OR=2.26 and the seroprevalence of 48% in background population, accordingly, we end up with PAR=37% (Figure 3). The result indicates that a vaccine cocktail combination of CBV1 and CBV2 is most interesting. These two viruses can lead to a product having a major effect to prevent the onset of the type 1 diabetes.

Altogether, the results indicate that CBV2 is also a risk virus associated with the pathogenesis of T1 D and therefore useful in the development of preventive vaccine.

## Claims

1. Live attenuated coxsackie B virus selected from the group consisting of CBV3 and CBV6 for use in preventing or treating type 1 diabetes.

2. The live attenuated coxsackie B virus of claim 1, wherein the use comprises vaccinating a subject with the live attenuated CBV3 or CBV6, and further with an inactivated diabetogenic enterovirus or a component thereof.

3. The live attenuated coxsackie B virus of claim 2, wherein the diabetogenic enterovirus is selected from the group consisting of CBV1, CBV2 and CAV16.

4. The live attenuated coxsackie B virus of claim 2, wherein the diabetogenic virus does not comprise coxsackie virus CBV3 nor CBV6, or any component thereof.

5. The live attenuated coxsackie B virus of claim 2, wherein the component of the diabetogenic enterovirus is a subunit or a nucleic acid of said virus.

6. The live attenuated coxsackie B virus of claim 1, wherein the use comprises vaccinating a subject with said live attenuated CBV3 or CBV6 and further administering antibodies against a diabetogenic virus to the subject.

7. The live attenuated coxsackie B virus of claim 6, wherein said antibodies do not comprise antibodies against CBV3 or CBV6.

8. Vaccine comprising live attenuated coxsackie B virus selected from the group consisting of CBV3 and CBV6.

9. The vaccine of claim 8 comprising live attenuated CBV3 and CBV6, and a pharmaceutically acceptable excipient and adjuvant.

10. The vaccine of claim 8 or 9, further comprising inactivated diabetogenic enterovirus, a component thereof, or an antibody thereto.

11. Method of preventing or treating type 1 diabetes in a subject in need thereof, comprising vaccinating the subject with at least one live attenuated coxsackie B virus selected from the group consisting of CBV3 and CBV6.

12. Method of inducing an immune response against a diabetogenic enterovirus in a subject in need thereof, comprising vaccinating the subject with at least one live attenuated coxsackie B virus selected from the group consisting of CBV3 and CBV6.

13. Method of eliminating the diabetogenic effect of an enterovirus in a subject in need thereof, comprising vaccinating the subject with at least one live attenuated coxsackie B virus selected from the group consisting of CBV3 and CBV6.
